# EUROPEAN PATENT APPLICATION

(11) **EP 2 044 898 A1**
(43) Date of publication of application: **08.04.2009**
(21) Application number: 08253234.2
(22) Date of filing: 03.10.2008
(51) Int. Cl.: A61B 17/34, A61B 17/00

(54) **Surgical portal with foam and fabric composite seal assembly**

(30) Priority: 05.10.2007 US 997845 P
(71) Applicant: Tyco Healthcare Group LP, North Haven, CT 06473 (US)
(72) Inventor: Izzo, Steven L., Naugatuck, CT 06770 (US); Bettuchi, Michael J., Middletown, CT 06457 (US); Schiretz, Frank R., Jr., Middletown, CT 06457 (US)
(74) Representative: Lloyd, John Scott

(57) **Abstract**

A surgical portal assembly includes a portal adapted to provide access to underlying tissue and having a longitudinal opening extending along a longitudinal axis of the portal and a seal. The seal includes internal surfaces having a passage for reception and passage of a surgical object in substantial sealed relation therewith and defines a seal axis. The seal includes a foam segment comprising a foam material and a fabric segment comprising a fabric material and being mounted relative to the foam segment. The fabric segment may be disposed adjacent one of the proximal and distal surfaces of the foam segment. The fabric segment may include a fabric layer which is in juxtaposed relation with the one of the proximal and distal surfaces of the foam segment. The fabric layer may include slots therethrough to facilitate passage of the surgical object through the seal. The slots may be arranged to extend radially outwardly relative to the seal axis.

## Description

### CROSS REFERENCE TO RELATED APPLICATION

The present application claims the benefit of and priority to U.S. Provisional Application Serial No. 60/997,845 filed on October 5, 2007, the entire contents of which are incorporated herein by reference.

### BACKGROUND

### 1. Field of the Disclosure

The present disclosure relates to surgical devices and, more particularly, relates to a surgical portal apparatus incorporating a seal assembly adapted for use during a minimally invasive, e.g., a laparoscopic, surgical procedure.

### 2. Description of the Related Art

Minimally invasive surgical procedures including both endoscopic and laparoscopic procedures permit surgery to be performed on organs, tissues and vessels far removed from an opening within the tissue. Laparoscopic and endoscopic procedures generally require that any instrumentation inserted into the body be sealed, e.g., provisions may be made to ensure that gases do not enter or exit the body through the incision as, for example, in surgical procedures in which the surgical region is insufflated. These procedures typically employ surgical instruments which are introduced into the body through a cannula. The cannula has a seal assembly associated therewith. The seal assembly is intended to form a substantially fluid tight seal about the instrument to preserve the integrity of the established pneumoperitoneum.

### SUMMARY

The present disclosure is directed to further improvements in seal assemblies for use with portal apparatii during a surgical procedure. In accordance with one embodiment, a surgical portal assembly includes a portal adapted to provide access to underlying tissue and having a longitudinal opening extending along a longitudinal axis of the portal and a seal. The seal includes internal surfaces having a passage for reception and passage of a surgical object in substantial sealed relation therewith and defines a seal axis. The seal includes a foam segment comprising a foam material and a fabric segment comprising a fabric material and being mounted relative to the foam segment. The fabric segment may be disposed adjacent one of the proximal and distal surfaces of the foam segment. The fabric segment may include a fabric layer which is in juxtaposed relation with the one of the proximal and distal surfaces of the foam segment. The fabric layer may include slots therethrough to facilitate passage of the surgical object through the seal. The slots may be arranged to extend radially outwardly relative to the seal axis.

The seal may have first and second fabric layers mounted in juxtaposed relation with respective proximal and distal surfaces of the foam segment. Alternatively, the seal may further include an elastomeric segment comprising an elastomeric material having less elasticity than the foam material of the foam segment. The elastomeric segment is mounted in juxtaposed relation to the fabric layer. The seal may include, from proximal to distal, the elastomeric segment, the fabric layer and the foam segment. A second layer of fabric may be mounted adjacent the distal surface of the foam segment and a third layer of fabric may be mounted adjacent a proximal surface of the elastomeric segment.

The seal may include an outer seal area and an inner seal area. The inner seal area generally tapers in a distal direction to define a general funnel configuration or a general sloped configuration which is adapted to facilitate insertion of the surgical object and minimize potential of inversion of the inner seal area upon withdrawal of the surgical object.

The portal may include a portal housing and a portal sleeve extending from the portal housing. In this embodiment, the seal may include a substantially annular retention member which is received within a corresponding annular recess of the portal housing to assist in mounting the seal within the portal housing.

### BRIEF DESCRIPTION OF THE DRAWINGS

Preferred embodiments of the present disclosure will be better appreciated by reference to the drawings wherein:
**FIG. 1** is a perspective views of a portal system in the form of a cannula assembly and a seal assembly in accordance with the principles of the present disclosure;
**FIG. 2** is a side plan view of the portal system of **FIG. 1**;
**FIG. 3** is a side cross-sectional view of the portal system;
**FIG. 4** is an enlarged view of the area of detail depicted in **FIG. 3**;
**FIG. 5** is a perspective view illustrating the seal assembly detached from the cannula assembly;
**FIG. 6** is a perspective view with parts separated of the seal assembly illustrating the seal housing, the object seal and the locking ring;
**FIGS. 7A**, **7B** and **7C** are respective plan, perspective and side-cross-sectional views of the object seal;
**FIG. 7D** is a perspective view with parts illustrating the components of the object seal;
**FIGS. 8A**, **8B** and 8C are respective front, side and rear plan views of the locking ring;
**FIGS**. **9A-9E** are views illustrating a sequence of assembling the seal components of the seal assembly; and
**FIG. 10** is a flow chart illustrating a method for performing a surgical task with the portal system of **FIG. 1**.

### DETAILED DESCRIPTION

The portal system of the present disclosure incorporates a seal assembly which, either alone or in combination with a seal internal to a cannula assembly, provides a substantial seal between a body cavity of a patient and the outside atmosphere before, during and after insertion of an object through the cannula assembly. Moreover, the seal assembly is capable of accommodating objects of varying diameters, e.g., instruments from about **4.5** mm to about **15** mm, by providing a gas tight seal with each instrument when inserted. The flexibility of the seal assembly greatly facilitates endoscopic surgery where a variety of instruments having differing diameters are often needed during a single surgical procedure.

The seal assembly contemplates the introduction and manipulation of various types of instrumentation adapted for insertion through a trocar and/or cannula assembly while maintaining a fluid tight interface about the instrumentation to prevent gas and/or fluid leakage from the established pneumoperitoneum so as to preserve the atmospheric integrity of a surgical procedure. Specifically, the seal assembly accommodates angular manipulation of the surgical instrument relative to the seal axis. This feature of the seal assembly desirably minimizes the entry and exit of gases and/or fluids to/from the body cavity. Examples of instrumentation include clip appliers, graspers, dissectors, retractors, staplers, laser probes, photographic devices, endoscopes and laparoscopes, tubes, and the like. Such instruments will be collectively referred to herein as "instruments or instrumentation".

The seal assembly may be a component of a portal system adapted to provide access to an underlying site. The seal assembly may be readily incorporated into a portal, such as a conventional trocar device or cannula, and provides the device with sealing capability about an inserted instrument.

The seal assembly may also be adapted to receive and form a seal about a physician's arm or hand during a hand-assisted laparoscopic procedure. In this application, the seal assembly is a component of an access member which is introduced within the body to provide access to underlying tissue in, e.g., the abdominal cavity.

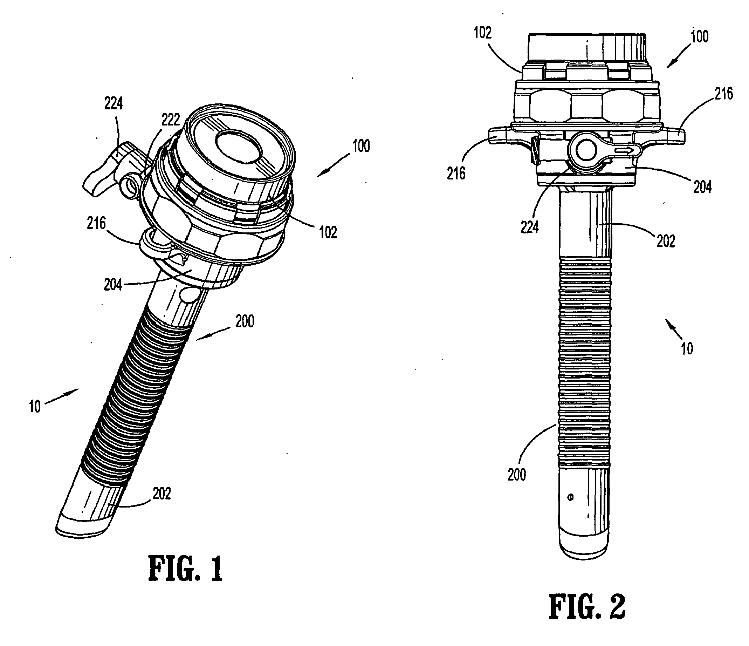

Referring now to the drawings, in which like reference numerals identify identical or substantially similar parts throughout the several views, **FIGS. 1-2** illustrate a portal system **10** of the present disclosure incorporating seal assembly 100 mounted to an access device such as cannula assembly **200**. Cannula assembly **200** may be any portal member suitable for the intended purpose of accessing a body cavity and typically defines a passageway permitting introduction of instruments therethrough. Cannula assembly **200** is particularly adapted for use in laparoscopic surgery where the peritoneal cavity is insufflated with a suitable gas, e.g., CO₂, to raise the cavity wall from the internal organs therein. Cannula assembly **200** is typically used with an obturator assembly (not shown) which may be blunt, a non-bladed, or a sharp pointed instrument positionable within the passageway of the cannula assembly **200**. The obturator assembly is utilized to penetrate the abdominal wall or introduce the cannula assembly 200 through the abdominal wall, and then subsequently is removed from the cannula assembly **200** to permit introduction of the surgical instrumentation utilized to perform the procedure through the passageway.

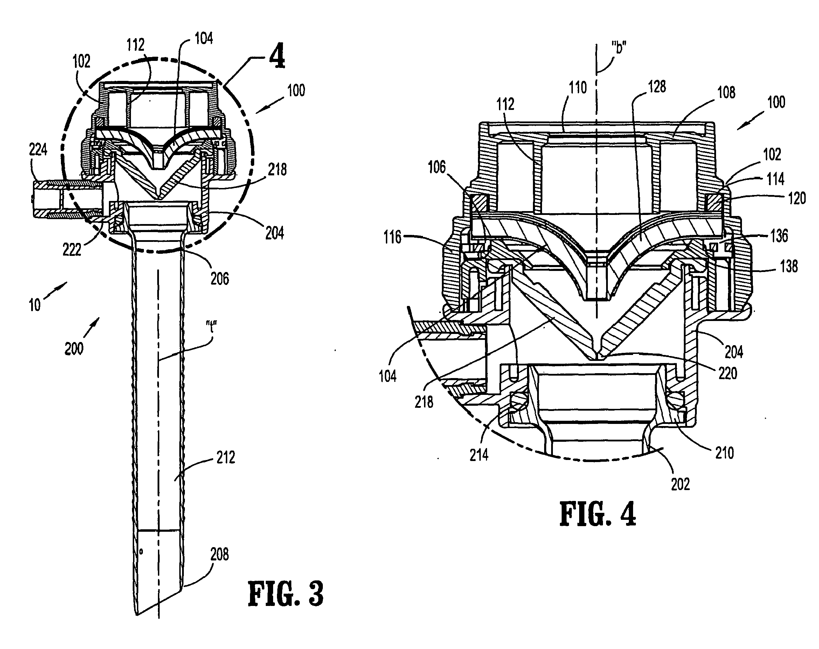

With respect to **FIGS. 3-4**, in conjunction with **FIGS. 1-2**, cannula assembly **200** includes cannula sleeve 202 and cannula housing 204 mounted to a proximal end of the cannula sleeve **202**. Cannula sleeve **202** defines a longitudinal axis "t" extending along the length of the cannula sleeve **202** and has proximal (or leading) and distal (or trailing) ends **206**, **208**. Mounted adjacent proximal end **206** of cannula sleeve **202** is sleeve flange **210**. Cannula sleeve **202** further defines an internal longitudinal passage **212** dimensioned to permit passage of surgical instrumentation. Cannula sleeve **202** may be formed of any suitable medical grade material, such as stainless steel or other rigid materials, including polymeric materials, such as polycarbonate, or the like. Cannula sleeve **202** may be transparent or opaque. The diameter of cannula sleeve **202** may vary, but, typically ranges from about **3.0** to about **18** mm. Cannula sleeve **202** may or may not include means for facilitating retention of the cannula sleeve **202** within tissue. Such means may include a plurality of locking elements or ribs such as, e.g., the locking arrangement disclosed in commonly assigned U.S. Patent Application Serial No. 11/170,824 to Smith filed June 30, 2005, the entire contents of the '824 disclosure being hereby incorporated by reference herein.

Cannula housing **204** may be connected to sleeve flange **210** of cannula sleeve 202 by conventional means including a bayonet coupling, a threaded connection, snap fit, ultrasonic welding or any other means envisioned by one skilled in the art including, e.g., adhesive means. Cannula assembly **200** may also incorporate O-ring seal **214** disposed between sleeve flange **210** and cannula housing **204** to assist in sealing the interior passages of cannula assembly **200**. Cannula housing **204** may be a single monolithically formed unit or composed of several components connected to each other through any of the aforementioned connection means. Cannula housing **204** further includes diametrically opposed housing grips **216** dimensioned and arranged for gripping engagement by the fingers of the clinician. Additionally or alternatively, suture anchors or filaments may extend from cannula housing **210**, e.g., from housing grips **216**, for attachment to the epidermis of the patient.

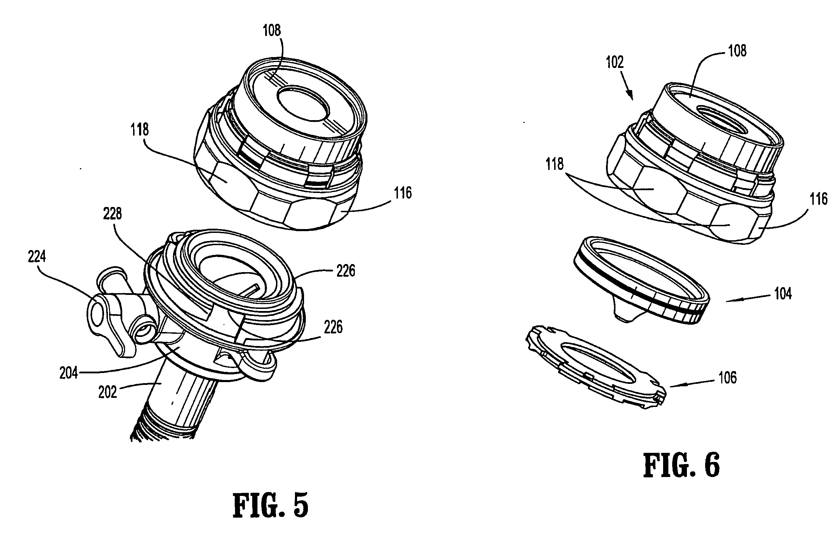

With reference to **FIGS. 1-5**, cannula housing 204 further includes valve **218**. Valve **218** may be a zero-closure valve such as a duck-bill valve having a slit **220** which is adapted to close in the absence of a surgical object and/or in response to insufflation gases of the pressurized cavity. In the alternative, valve **218** may be a gel seal, balloon valve, or a flapper valve. Cannula housing **204** may further include port **222** to which stop cock valve **224** is attached. Port **222** permits the introduction of insufflation gases though cannula sleeve **202** via opening of stop cock valve 224 to assist in maintaining the integrity of the pneumoperitoneum. Stop cock valve **224** may be any conventional valve. As best depicted in **FIG. 5**, cannula housing **204** further includes at least one, e.g., three, peripheral grooves **226**. Grooves **226** extend in an axial direction and are preferably equidistally spaced about the periphery of the cannula housing **204**. Grooves **226** assist in releasably mounting seal assembly **100** to cannula assembly **200**.

Referring now to **FIGS. 5-6**, in conjunction with **FIGS. 3-4**, seal assembly **100** will be discussed in detail. Seal assembly **100** includes seal housing, generally identified as reference numeral **102**, composite object seal **104** which is disposed within the seal housing **102** and internal locking element **106**. Seal housing **102** houses the sealing components of the assembly and defines the outer valve or seal body of the seal assembly **100**. Seal housing **102** defines central seal housing axis "b" which is preferably parallel to the axis "t" of cannula sleeve **202** and, more specifically, coincident with the axis "t" of the cannula sleeve **102** when seal assembly **100** is mounted to cannula assembly **200**. Seal housing **102** may be integrally or monolithically formed as a single unit or may incorporate multiple components which, when assembled together, form the seal housing **102**. In the illustrated embodiment, seal housing **102** is a single unit formed of a suitable polymeric material. Suitable polymeric materials include polycarbonates polystyrenes, ABS or any other material contemplated by one skilled in the art.

Seal housing **102** includes proximal end wall **108** defining central aperture **110** and internal annular collar **112** depending from the end wall **108** and coaxially arranged about seal housing axis "b". Central aperture **110** and annular collar **112** receive the surgical object and collectively define an internal dimension or diameter adapted to permit passage of relatively large sized instruments. Annular collar **112** also may limit the degree of lateral or offset movement of the surgical object, e.g., surgical instrument, relative to seal axis "b" by, defining an outer limit of movement of the instrument. Seal housing **102** further defines internal peripheral recess or channel **114** (**FIG. 4**) approximately adjacent the longitudinal midpoint of the seal housing **102** for receiving a component of object seal **104** as will be discussed.

Seal housing **102** further includes mounting collar 116 adjacent its distal end. Mounting collar **116** may be selectively releasably connectable to cannula housing **204** to cooperatively releasably couple seal assembly **100** to cannula assembly **200**. Various means for releasably securing or connecting mounting collar to cannula housing are envisioned including a bayonet coupling, snap-fit, frictional fit, tongue and groove arrangement, threaded arrangement, cam-lock mechanisms or the like. One methodology contemplated will be discussed in greater detail hereinbelow. Alternatively, seal housing **102** may be permanently secured to cannula housing **204**. Mounting collar **116** may have an irregular exterior surface to facilitate engagement by the clinician. In one embodiment, mounting collar includes an arrangement of spaced recesses **118** to assist in gripping of seal assembly **100**.

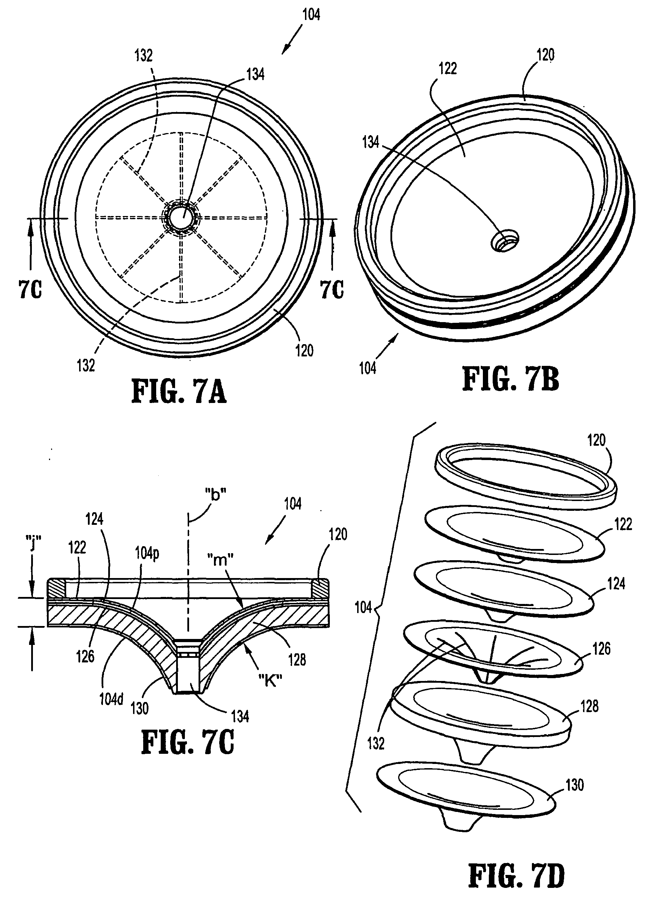

With particular reference to **FIGS. 7A-7D**, in view of **FIGS. 4** and **6**, composite object seal **104** will be discussed in detail. Object seal 104 includes from, proximal to distal, retention member **120**, first fabric segment **122**, elastomeric segment **124**, second fabric segment **126**, foam segment **128** and third fabric segment **130**. Retention member **120** is general annular or ring-like in shape and may be fabricated from a suitable biocompatible relatively rigid material such as polypropylene, nylon, ABS, polycarbonate, stainless steel, titanium or any other suitable material. Retention member **120** assists in mounting object seal **104** within seal housing **102** by its reception within peripheral channel **114** of the seal housing **102** (**FIG. 4**). First fabric segment **122**, second fabric segment **126** and third fabric segment **130** may each be in the form of disc-shaped layers. Fabric segments **122, 126, 130** generally enhance the structural integrity of object seal **104** by providing a support lattice or structure to encapsulate and support foam segment **128**. Fabric segment **122**,**130** also may prevent foam segment **128** of object seal **104** from contact with the instrument during insertion and possibly withdrawal of the instrument through object seal **104**. Fabric segments **122, 126, 130** also enhance seal durability and may reduce object or instrument insertion forces. A suitable fabric material for each of fabric segments **122, 126, 130** includes a SPANDEX^{™} material containing, e.g., 20% LYCRA^{™} which is commercially available from Milliken of South Carolina. The fabric may comprise a woven, knitted, braided, or non-woven material of polymeric materials. Other fabric materials are also envisioned. For example, a synthetic material such as nylon, Kevlar (Trademark of E.I. DuPont de Nemours and Company) or any other material that will expand and compress about an instrument inserted therethrough is envisioned. In addition, the fabric may be coated, e.g., on its interior with urethane, silicon or other flexible lubricious materials to facilitate passage of an instrument or other object, through the seal.

Each fabric segment **122, 126, 130** may define an aperture, opening or passage to permit passage of the surgical object. Single or multiple intersecting slits within any one or more of fabric segments **122, 126, 130** are also envisioned. For example, second fabric segment **126** may define at least one, possibly, a plurality of slits **132** extending outwardly from seal axis "b". In one embodiment, slits **132** are substantially linear and extend radially outwardly relative to seal axis "b". Other arrangements are envisioned including non-linear slits, serpenditious slits, intersecting slits. Slits **132** may be equidistally and radial spaced about seal axis. Slits **132** may assist in reducing insertion and withdrawal forces needed to advance the object into the surgical site by reducing radial constriction of the inner areas of fabric segment **130** about the object.

With continued reference to **FIGS. 7A-7D**, elastomeric segment **124** in fabricated from a suitable elastomeric or thermoplastic polymer which may exhibit less elasticity than the elasticity of the material of foam segment **128**. Elastomeric segment **124** provides protection to foam segment **128** by minimizing the potential of puncture with the surgical object, e.g., the instrument during insertion. Elastomeric segment **128** may be fabricated from a polyisoprene; however, other elastomers are also envisioned including neoprene, silicone, butyl, nitrile and Buna-N.

Foam segment **128** is fabricated from a foam material (closed cell or open cell) such as, in one embodiment, a thermoplastic material comprising a foaming agent. Foam segment **128** may be the primary sealing component about the surgical object. In one embodiment, foam segment 128 is fabricated from a polyisoprene impregnated or injected with a foaming agent including, e.g., CELOGEN^{™}, EXPANDEX^{™}, and OPEX^{™} chemical foaming agents. Foam segment **128** has sufficient elasticity to bend and deform about the inserted instrument while conforming about the outer dimensioning of the object, e.g., instrument, thereby establishing a fluid tight seal about the object. Foam segment **128** is sufficiently compliant to absorb off axis motion of the instrument. Moreover, the compliant characteristics of foam segment **128** may substantially minimize the formation of a gap around the instrument during off-set manipulation of the instrument. The presence of a gap would otherwise permit the undesired release of gases from the underlying pneumoperitioneum.

In the assembled condition of object seal **104**, best depicted in **FIG. 7C**, the object seal **104** defines a generally tapered or funneled profile whereby the inner area of the object seal **104** slopes at an oblique angle with respect to the seal axis "b". The funneled characteristic may assist in guiding the instrument toward central aperture 134 during initial introduction of the instrument. The funneled characteristic also may substantially minimize the potential of inversion of object seal 104 during withdrawal of the instrument. More particular, object seal 104 defines proximal seal surface 104p adjacent fabric segment **122** and distal seal surface 104p adjacent fabric segment 130 defining an arcuate, parabolic or hyperbolic profile. In one embodiment, proximal seal surface **104p** may define a surface portion having a radius of curvature "m" ranging from about .**540** inches to about .**620** inches, and distal seal surface **104d** may define a surface portion having a radius of curvature "k" ranging from about .**430** inches to about .500 inches. Other dimensioning and radii of curvatures are also envisioned. The axial length "j" of object seal **104** ranges from about .47 inches to about .53 inches (excluding retention member 120). This overall dimensioning effected through the sloped arrangement provides an exaggerated funneled profile to object seal 104, which, facilitates directing or funneling of the surgical object through object seal 104 and, also, minimizes the potential of inversion of the object seal 104 during withdrawal of the object.

Object seal **104** may be manufactured via conventional means. In one method, fabric segments **122, 126, 130,** elastomeric segment **124** and foam segment **128** may be compression molded while the elastomeric material of the elastomeric segment **124** and/or the foam material of the foam segment **128** is subjected to heat to at least partially embed the fabric material of fabric segments **122, 126, 130** into the elastomeric and/or foam segments **124, 128.** Alternatively, the components of object seal **104** may be attached with adhesives, cements, or the like. The methodologies for attaching fabric segments **122, 126, 130** to one or both of elastomeric and foam segments **124, 128** as disclosed in certain embodiments of the U.S. Patent. No. 6,702,787 to Racenet also may be utilized. The entire disclosure of U.S. Patent No. 6,702,787 to Racenet is hereby incorporated by reference herein. Another method for fabricating seal **104** is disclosed in (H-US-**00875**), the entire contents of which are hereby incorporated by reference herein. Once object seal **104** is assembled or manufactured, central seal aperture **134** may be punched through the composite materials with a die punch or made via a molding process which provides the seal aperture **134**. Alternatively, the respective components of object seal **104** may be provided with cooperative aperture or slits and then assembled via any of the aforementioned methodologies.

In the alternative, object seal **104** may be a substantially flat or planar septum seal having an aperture, slit or the like. Object seal **104** also may comprise a gel composition or very-soft thermoplastic elastomer in addition to, or in lieu of, foam segment **128**. Gels and soft thermoplastic materials contemplated for use are known under the trade names VERSAFLEX^{™}, FLEXPLAST^{™}, DYANFLEX^{™} and MONPRENE^{™}. Other suitable materials include soft silicone and polyurethane composites. These materials may be adapted to be sufficiently pliable.

Object seal **104** may incorporate a lubricant or a therapeutic or pharmacological agent. Suitable lubricants include a coating of hydrocyclosiloxane prepared by plasma polymerization process. Such a coating is disclosed in U.S. Pat. No. 5,463,010 to Hu et al., the entire contents of which is hereby incorporated by reference. Examples of therapeutic or pharmacological agents include antimicrobials, antibacterials, hemostatic, moisture-providing agents, such as saline, healing agents, lubricious agents, analgesics, antiseptics, growth factors, and/or anti-inflammatory agents.

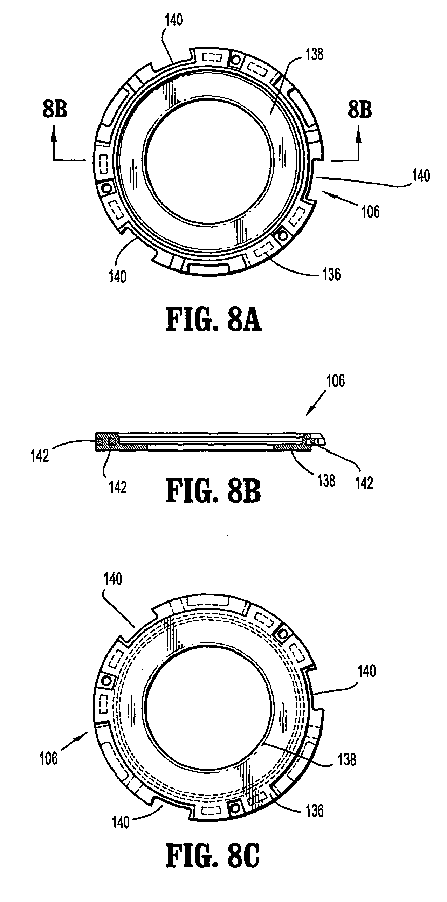

Referring now to **FIGS. 8A-8C**, in conjunction with **FIGS. 4** and **6**, locking ring **106** of seal assembly **100** will be discussed. Locking ring **106** serves a dual function of securing object seal **104** within seal housing **102** and providing an internal seal within the passageway of the assembled seal and cannula assemblies **100**, **200**. Locking ring **106** may be fabricated from a material exhibiting some resiliency including any elastomeric material hereinabove described. Locking ring **106** includes outer segment **136** and internal gasket segment **138**. Outer segment **136** defines a plurality (e.g., three) peripheral recesses **140** equidistally spaced about the periphery. Recesses **140** assist in mounting locking ring **106** within seal housing **102**. Internal gasket segment **138** of locking ring **106** may contact distal seal surface **104d** of object seal **104** in the assembled condition of seal assembly **100**. Outer segment **136** and internal gasket segment **138** may be monolithically formed or may consist of separate components received to each other by conventional means. Internal gasket segment **138** may be more elastic than outer segment **136**. Outer segment **136** may incorporate first and second O-ring seals **142** within annular recesses of the outer segment to assist in sealing the internal opening of seal housing **102**.

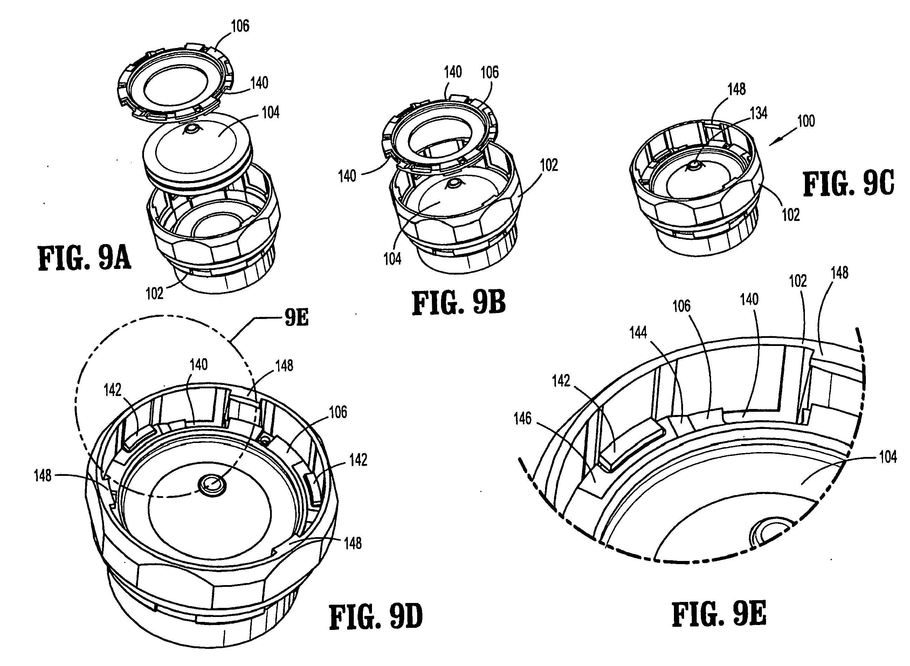

The assembly of seal assembly **100** will now be discussed. With reference to **FIGS. 9A-9F**, in conjunction with **FIG. 4**, object seal **104** is aligned with the internal area of seal housing **102** and advanced within the seal housing **102** whereby retention member **120** is received within channel **114** of seal housing **102** (see also **FIG. 4**). Locking ring 106 is thereafter positioned whereby peripheral recesses **140** of the locking ring 106 are aligned with mounting tabs **142** extending radially inwardly from the interior wall of seal housing **102**. In particular, as best depicted in **FIGS. 9D-9E**, mounting tabs **142** are disposed within the interior of seal housing **102**. At least two, e.g., three, mounting tabs **142** and associated peripheral recesses **140** of locking ring **106** are envisioned. Locking ring **106** is advanced into seal housing **102** with mounting tabs **142** being received within peripheral recesses **140** of the locking ring **106**. Thereafter, locking ring **106** is rotated relative to seal housing **102** to cause mounting tabs **142** of seal housing **102** to ride along peripheral surfaces on the underside of the locking ring **106** to be out of alignment with respective peripheral recesses **140**. In this arrangement as depicted in **FIG. 9E****,** mounting tabs **140** are secured against the underside of locking ring **106**. It is envisioned that mounting tabs **140** may initially ride along inclined or cam surfaces **144** provided on the underside of locking ring **106** and adjacent recesses **140** to draw the locking ring **106** against object seal **104**. It is further envisioned that the underside of locking ring **106** may have locking recesses **146** formed therein to assist in securing locking tabs **106** relative to seal housing **102**. **FIG. 9D** illustrates the assembled seal assembly **100**.

Seal assembly **100** may be associated with, or joined to, cannula assembly **200** in a variety of ways. In a preferred embodiment, seal housing **102** of seal assembly **100** and cannula housing **204** of cannula assembly **200** are adapted to detachably engage each other, e.g., through a bayonet lock, threaded attachment, latching attachment, or like mechanical means. In one preferred arrangement, seal housing **102** includes at least two, preferably, three housing locking detents **148** as best depicted in **FIGS**. **9D-9E****.** Locking detents **148** are aligned with and subsequently received within, axial recesses of cannula housing **204** (**FIG. 5**) during mounting of seal assembly **100** to cannula assembly **200**. In one embodiment, seal housing **102** and cannula housing **104** are rotated relative to each other to position housing locking detents beneath on annular ledge **228** defined within the cannula housing **104** to receive the components. Alternatively, seal housing **102** and cannula housing **104** may be releasably secured to each other via a friction fit or the like. Seal assembly may be mounted to cannula assembly **100** before, during, or after, application of cannula assembly **200** within the operative site.

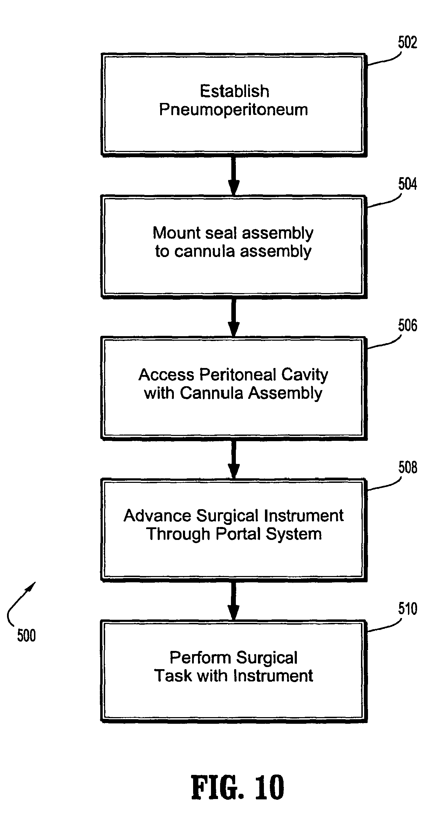

**FIG. 10** is a flow chart **500** illustrating the use of seal assembly **100** and cannula assembly **200** in connection with the performance of a surgical task during a laparoscopic procedure. The peritoneal cavity is insufflated to establish the pneumoperitonum (Step **502**). Seal assembly **100** is mounted to cannula assembly **200** (step **504**) as discussed hereinabove. The assembled portal system **10** is introduced into an insufflated abdominal cavity typically utilizing a sharp or non-blade trocar obturator to access the cavity (step **506**) and the obturator is removed. An instrument may be advanced through portal system **10** (step **508**) by inserting the instrument into seal assembly **100** through object seal **104** whereby the portions defining aperture **134** of the object seal **104** stretch to accommodate the instrument in substantial sealed relation therewith. The instrument is distally passed through valve **218** and into the body cavity. The desired surgical task is performed with the instrument (**510**). During manipulation of the instrument, at least the foam material of foam segment **128** conforms about the instrument to prevent formation of any gaps on opposed sides of the instrument. Fabric segments **122, 126, 130**, and elastomeric segment **124** assist in supporting and preserving the integrity of foam segment **128** during insertion, manipulation and withdrawal of the surgical instrument.

It will be understood that various modifications and changes in form and detail may be made to the embodiments of the present disclosure without departing from the spirit and scope of the invention. Therefore, the above description should not be construed as limiting the invention but merely as exemplifications of preferred embodiments thereof. Those skilled in the art will envision other modifications within the scope and spirit of the present invention as defined by the claims appended hereto. Having thus described the invention with the details and particularly7 required by the patent laws, what is claimed and desired protected is set forth in the appended claims.

A surgical portal assembly includes a portal adapted to provide access to underlying tissue and having a longitudinal opening extending along a longitudinal axis of the portal and a seal. The seal includes internal surfaces having a passage for reception and a passage of a surgical objection in substantial sealed relation therewith and defines a seal axis. The seal includes a foam segment comprising a foam material and a fabric segment comprising a fabric material and being mounted relative to the foam segment, the fabric segment may be disposed adjacent one of the proximal and distal surfaces of the foam segment. The fabric segment may include a fabric layer which is in juxtaposed relation with the one of the proximal and distal surfaces of the foam segment. The fabric layer may include slots therethrough to facilitate passage of the surgical object through the seal. The slots may be arranged to extend radially outwardly relative to the seal axis.

## Claims

1. A surgical portal assembly, which comprises:
a portal adapted to provide access to underlying tissue and having a longitudinal opening extending along a longitudinal axis of the portal, and defining proximal and distal ends; and
a seal including internal surfaces having a passage for reception and passage of a surgical object in substantial sealed relation therewith and defining a seal axis, the seal including a foam segment comprising a foam material, and a fabric segment comprising a fabric material and being mounted relative to the foam segment.

2. The surgical portal assembly according to claim 1 wherein the fabric segment is disposed adjacent one of the proximal and distal surfaces of the foam segment.

3. The surgical portal assembly according to claim 2 wherein the fabric segment includes a fabric layer, the fabric layer in juxtaposed relation with the one of the proximal and distal surfaces of the foam segment.

4. The surgical portal assembly according to claim 3 wherein the fabric layer includes slots therethrough to facilitate passage of the surgical object through the seal.

5. The surgical portal assembly according to claim 4 wherein the slots are arranged to extend radially outwardly relative to the seal axis.

6. The surgical portal assembly according to claim 3 including first and second fabric layers mounted in juxtaposed relation with respective proximal and distal surfaces of the foam segment.

7. The surgical portal assembly according to claim 3 wherein the seal further includes an elastomeric segment comprising an elastomeric material having less elasticity than the foam material of the foam segment.

8. The surgical portal assembly according to claim 7 wherein the elastomeric segment is mounted in juxtaposed relation to the fabric layer.

9. The surgical portal assembly according to claim 8 wherein the seal includes, from proximal to distal, the elastomeric segment, the fabric layer and the foam segment.

10. The surgical portal assembly according to claim 9 further including a second layer of fabric mounted adjacent the distal surface of the foam segment.

11. The surgical portal assembly according to claim 10 further including a third layer of fabric mounted adjacent a proximal surface of the elastomeric segment.

12. The surgical portal assembly according to claim 3 wherein the seal includes an outer seal area and an inner seal area, the inner seal area generally tapering in a distal direction to define a general funnel configuration.

13. The surgical portal assembly according to claim 3 wherein the seal includes an outer seal area and an inner seal area, the inner seal area defining a general sloped portion to facilitate insertion of the surgical object and minimize potential of inversion of the inner seal area upon withdrawal of the surgical object.

14. The surgical portal assembly according to claim 2 wherein the portal includes a portal housing and a portal sleeve extending from the portal housing.

15. A surgical portal assembly according to claim 14 wherein the seal includes a substantially annular retention member, the retention member being received within a corresponding annular recess of the portal housing to assist in mounting the seal within the portal housing.
